# EUROPEAN PATENT SPECIFICATION

(11) **EP 3 723 496 B1**
(45) Date of publication and mention of the grant of the patent: **31.05.2023**
(21) Application number: 18812200.6
(22) Date of filing: 11.12.2018
(51) Int. Cl.: C07K 14/47, C07K 14/79, C07K 19/00, C12N 9/36, A23J 3/08, A23J 3/10, A23L 33/19, A23K 20/147, A23L 33/00, A61K 38/00

(54) **COMPLEXES OF HIGH ISOELECTRIC POINT PROTEINS WITH CASEIN**
KOMPLEXE VON HOHEN ISOELEKTRISCHEN PUNKT-PROTEINEN MIT CASEIN
COMPLEXES DE PROTÉINES À POINT ISO-ÉLECTRIQUE ÉLEVÉ AVEC DE LA CASÉINE

(30) Priority: 12.12.2017 EP 17206619
(43) Date of publication of application: 21.10.2020
(73) Proprietor: Société des Produits Nestlé S.A., 1800 Vevey (CH)
(72) Inventor: GEE, Vivian,Ling, Cork P51 K0Y3 (IE); O'REGAN, Jonathan, KILLARNEY, Kerry V93E1T2 (IE); WALSHE, Emma,Joanne, Cork P61Y030 (IE)
(74) Representative: Gagliardi, Tatiana
(86) International application number: PCT/EP2018/084311
(87) International publication number: WO 2019/115507

(56) References cited:
- AU-A- 2 610 595
- PL-B1- 230 316
- US-A1- 2022 055 042
- LI QUANYANG ET AL: "Formation of lactoferrin/sodium caseinate complexes and their adsorption behaviour at the air/water interface", FOOD CHEMISTRY, ELSEVIER LTD, NL, vol. 232, 13 April 2017 (2017-04-13), pages 697-703, XP085001578, ISSN: 0308-8146, DOI: 10.1016/J.FOODCHEM.2017.04.072
- PAN ET AL: "Self-assembly of @b-casein and lysozyme", JOURNAL OF COLLOID AND INTERFACE SCIENCE, ACADEMIC PRESS,INC, US, vol. 316, no. 2, 5 November 2007 (2007-11-05), pages 405-412, XP022328632, ISSN: 0021-9797, DOI: 10.1016/J.JCIS.2007.09.007
- AHMED O. ELZOGHBY ET AL: "Casein-based formulations as promising controlled release drug delivery systems", JOURNAL OF CONTROLLED RELEASE, vol. 153, no. 3, 1 August 2011 (2011-08-01), pages 206-216, XP055113658, ISSN: 0168-3659, DOI: 10.1016/j.jconrel.2011.02.010
- KANYSHKOVA T G ET AL: "Lactoferrin and its biological functions", BIOCHEMISTRY, MAIK NAUKA - INTERPERIODICA, RU, vol. 66, no. 1, 1 January 2001 (2001-01-01), pages 5-13, XP002558215, ISSN: 0006-2979
- Anonymous: "Lactoferrin ? A Balanced Prebiotic in the Treatment of Dysbiosis ? Naturopathic Doctor News and Review", , 19 January 2009 (2009-01-19), XP055537767, Retrieved from the Internet: URL:https://ndnr.com/gastrointestinal/lact oferrin-a-balanced-prebiotic-in-the-treatm ent-of-dysbiosis/ [retrieved on 2018-12-21]
- CHEN YUE ET AL: "Lactoferrin Promotes Early Neurodevelopment and Cognition in Postnatal Piglets by Upregulating the BDNF Signaling Pathway and Polysialylation", MOLECULAR NEUROBIOLOGY, SPRINGER US, NEW YORK, vol. 52, no. 1, 23 August 2014 (2014-08-23), pages 256-269, XP036144595, ISSN: 0893-7648, DOI: 10.1007/S12035-014-8856-9 [retrieved on 2014-08-23]

## Description

### Field of the invention

The present invention provides a process for producing complexes of high isoelectric point proteins with casein comprising a specific sequence of heating, cooling and holding steps. Also provided are the complexes obtained by such process and their use, in particular their use in methods of therapy.

### Background of the invention

Several bioactive proteins such as lactoferrin, lysozyme or lactadherin have been identified as having beneficial health benefits and thus it has been attempted to use such proteins in nutritional products or pharmaceutical products. Such proteins are however very sensitive to harsh treatments, such as exposure to high heat, and it proved difficult to include such proteins in industrial products. There is therefore a need to develop stabilized forms of such proteins.

Lactoferrin, for example, is a component of human milk. It is also present in products such as infant formula, but at much lower levels than in human breast milk. It has been therefore previously described to complement infant formula with lactoferrin from bovine origin. Bovine lactoferrin (bLf) is an iron binding glycoprotein of approximately 77 kDa and consists of a single polypeptide chain of about 700 amino acids. Human lactoferrin is a glycoprotein with a molecular weight of 80 kDa, which shows high affinity for iron. The sequence homology between human and Bovine Lf is about 70% and the 3-D structure of both are very similar but not identical.

Lf is a strongly cationic protein with a high isoelectric point (pl) of approximately 8.9. At neutral pH (~pH7) Lf is known to bind to other anionic proteins such as β-lactoglobulin, casein, and albumin. For this reason, when lactoferrin is added during wet mixing of an infant formula blend, the proteins would under unfolding denaturation and refolding under the effect of temperature and pH, resulting in protein instability and increases in viscosity. This phenomenon makes it very complicated to add lactoferrin in wet (i.e. before drying) in a product such as infant formula.

Therefore, it has been described to add lactoferrin into products such as infant formula in solid form, by dry-mixing after the base powder of the product has been produced. Such process is however challenging because lactoferrin added in solid form needs to be sterile. To achieve the required level of sterility, lactoferrin would need to be subjected to sterilization techniques. Most sterilization techniques involve the use of high heat, which may lead to denaturation of the lactoferrin. Other serilization techniques, such as membrane filtration are available but may be costly and require specific equipment. Also, addition of the sterile lactoferrin in the final product requires specific and precise aseptic dosing equipment.

Therefore, to overcome these problems, it would be highly desirable to develop alternative forms of lactoferrin, allowing addition of lactoferrin together with the other ingredients of an infant formula in the wet mix and aseptic processing or spray-drying in the infant formula composition.

As an alternative to addition in native form, it has been described to add lactoferrin to edible products in the form of complexes. WO2012/045801 describes complex coacervates comprising lactoferrin and at least one other protein with an isoelectric point lower than pH 7.0, which can be used to delay protein digestion and to improve the metabolic responses, gut inflammatory response, satiety and food intake in human. This document however does not address the problem of stabilizing lactoferrin during processing of a product like an infant formula and in particular does not indicate the described coacervates would be sufficiently stable to be added in in the wet mix in the manufacture of an infant formula.

Oppositely charged proteins in solution (which are colloids) interact to form complexes spontaneously under specific pH, ionic strength and stoichiometry. Such complexes can remain soluble or cause liquid-liquid phase separation in the solution. In the latter case complex coacervates are formed. "The phase more concentrated in colloid component is the coacervate, and the other phase is the equilibrium solution". (IUPAC, 1997) Both phases remain in equilibrium and are therefore said to be incompatible. Both the soluble complexes and the coacervates are very sensitive to the properties of the medium and are generally reversible under the effect of pH change, ionic strength or temperature and therefore may be destroyed during processing of a food product. Such reversibility of protein-protein complexes has been described in the literature. For example Anema et al.; Coacervates of lysozyme and β-casein; Journal of Colloid and Interface Science 398 (2013): 255-261 reports that complexes of lactoferrin or lysozyme and β-casein are reversible, namely under the effect of pH change and ionic strength (e.g. addition of NaCl).

It has been described to make complex coacervates more robust by using cross-linking agents. Addition of cross-linking agents would however be undesirable in products for sensitive consumers like infants and young children, wherein the ingredients that can be used are limited by very stringent regulations.

In WO2012/045801 complex coacervates of lactoferrin and a protein having an isoelectric point below 7 are induced by pH. However, the complexes such as those described in this document are not stable enough to resist harsh treatment such as high heat, which would make them unsuitable to be processed with the other ingredients of a product, such as an infant formula.

Also, Anema et al.; Co-acervates of lactoferrin and caseins; Soft Matter 8 (2012): 4471-4478, has studied the formation of coacervates of lactoferrin and caseins, namely β-casein, induced by adjusting the pH to 6.55 at different temperatures. However, this document does not address at all the question of the reversibility of the obtained coacervates, which may be damaged when subjected to harsh processing such as sterilization or drying.

Li Quanyang et al.; Formation of lactoferrin/sodium caseinate complexes and their adsorption behaviour at the air/water interface; Food Chemistry, 232 (2017); 697-703, describes a process involving heating followed by immediate cooling of a solution of sodium caseinate and lactoferrin. The process described in this document is disadvantageous in that it is unsuitable for forming complexes with micellar casein. It is desirable to provide a process allowing the formation of complexes with micellar casein as well.

Pan et al.; Self-assembly of β-casein and lysozyme; Journal of Colloid and Interface Science, 316(2) (2007), 405-412 describes a process of forming complexes of β-casein and lysozyme involving pH adjustment in suitable range extending between 4.0 and 6.0 or between 9.0 and 12.0, followed by heat treatment at 80°C for 30 minutes and cooling. The process leads to formation of nanoparticles that are unstable at pH 3.0 and 12.0 and aggregate in the presence of 0.15 M NaCl. Only grafting of dextran to β-casein through Maillard reaction stabilized the nanoparticles at neutral pH and salt solution. It is desirable to provide a process yielding more stables complexes able to resist such conditions without addition of further component such as dextran in the complex. This is in particular important in infant formula, wherein it is desirable to keep the list of ingredients as short as possible and wherein the regulatory constraints are very strict.

It is desirable to provide lactoferrin, and/or other sensitive proteins such as lysozymes, in a form which makes it sufficiently robust to undergo harsh treatment, such as heat sterilization or drying, thus avoiding the need of alternative sterilization and aseptic dosing methods, which make the manufacture of products with sensitive proteins like lactoferrin significantly more complicated and costly. It is further desired to be able to form complexes with all types of casein, including micellar casein.

The present invention advantageously solves the above-mentioned problems.

### Summary of the invention

In a first aspect, the invention provides a process for producing complexes of a protein having an isoelectric point of at least 7 with casein comprising the steps of:
a. providing a solution of a protein having an isoelectric point of at least 7 and casein at a pH in the range of 6.5 to 7.5;
b. ramping up the temperature from the denaturation temperature of the protein having an isoelectric point of 7 or more to a temperature in the range of 85 to 95°C;
c. holding the solution at a temperature of 85 to 95°C for at least 30 seconds; and
d. ramping down the temperature from 85-95°C to a temperature below 5°C over a period of at least 30 minutes
e. holding the solution at a temperature below 5°C for at least 15 minutes.

In a second aspect, the invention provides complexes of a protein having an isoelectric point of at least 7 with casein, obtainable or obtained by the process of the invention.

In a third aspect, the invention provides a product comprising the complexes of the invention.

In a fourth aspect the invention provides a process for producing a product of the invention.

In a fifth aspect, the invention provides the complexes of the invention for use in therapy.

In a fifth aspect, the invention relates to a complexes of the invention for use in a method of
a) preventing, reducing and/or treating infections;
b) modulating, promoting and/or supporting the immune response;
c) promoting and/or supporting the growth of beneficial gut flora;
d) promoting and/or supporting cognitive function;
e) promoting and/or supporting healthy growth of a infant or young child; or
f) promoting softer stools in an infant.

### Brief Description of the Drawings

**Figure 1****:** CLSM image of the coacervates obtained in Example 1. Still image taken through the Z-stack of the sample using the 63 objective lens. Coacervates appear as uniform spherical particles having a porous structure.
**Figure 2****:** A: CLSM image of the coacervates obtained in Example 1 after centrifugation. Image of the pellet after centrifugation, taken using the 20 objective lens. B: picture of the coacervate solution after the centrifugation. The coacervate sediment is clearly visible.
**Figure 3****:** CLSM image of the coacervates obtained in Example 1 before (A) and after (B) refrigeration for 24 hours using the 20 objective lens. Coacervates are clearly seen on both images, although the coacervates after one night of refrigeration are smaller.
**Figure 4****:** CLSM image of the composition obtained in Example 2 (comparative) using the 20 objective lens. No coacervates can be seen in the supernatant (A) and in the sediment (B).
**Figure 5****:** CLSM image of the composition obtained in Example 3 (comparative) using the 20 objective lens. No coacervates can be seen in the supernatant (A) and in the sediment (B).
**Figure 6****:** CLSM image of the composition obtained in Example 4 (comparative) using the 20 objective lens. No coacervates can be seen in the supernatant (A) and in the sediment (B).
**Figure 7****:** Light microscopy image of the coacervates of Example 5 after dilution of coacervates in SDS. Coacervates can still be seen as uniform spherical particles. Coacervates are enlarged 10 times.
**Figure 8****:** Light microscopy image of the coacervates of Example 5 after being subjected to high speed centrifugation. Coacervates can still be seen as uniform spherical particles. Coacervates are enlarged 10 times.
**Figure 9****:** Light microscopy image of the coacervates of Example 5 after dilution with water and adjustment of the pH to 3.5. Coacervates can still be seen as uniform spherical particles. Coacervates are enlarged 10 times.
**Figure 10****:** Light microscopy image of the coacervates of Example 5 after dilution with water and adjustment of the pH to 3.5 and subsequent heating at 70°C for 15 minutes. Coacervates can still be seen as uniform spherical particles. Coacervates are enlarged 10 times.
**Figure 11****:** Light microscopy image of the coacervates of Example 5 after dilution with water and adjustment of the pH to 11. Coacervates can still be seen as uniform spherical particles. Coacervates are enlarged 10 times.
**Figure 12****:** Light microscopy image of the coacervates of Example 5 after dilution with water and adjustment of the pH to 10 and subsequent heating at 70°C for 15 minutes. Coacervates can still be seen as uniform spherical particles. Coacervates are enlarged 10 times.
**Figure 13****:** Light microscopy image of the coacervates of Example 5 after dilution with 100 mM NaCl. Coacervates can still be seen as uniform spherical particles. Coacervates are enlarged 10 times (A) and 20 times (B) respectively.
**Figure 14****:** Light microscopy image of the coacervates of Example 6. Coacervates can be seen as uniform spherical particles. Coacervates are enlarged 10 times on the larger picture and a 4x zoom is provided in the smaller picture.

### Detailed description of the invention

### Definitions

As used herein, the following terms have the following meanings.

The term **"infant"** means a child under the age of 12 months.

The expression **"young child"** means a child aged between one and seven years.

The expression **"nutritional composition"** means a composition which nourishes a subject. This nutritional composition is usually to be taken orally or intravenously, and it usually includes a lipid or fat source and a protein source.

In a particular embodiment the composition of the present invention is a hypoallergenic nutritional composition. The expression **"hypoallergenic nutritional composition"** means a nutritional composition which is unlikely to cause allergic reactions.

In a particular embodiment the composition of the present invention is a "synthetic nutritional composition". The expression **"synthetic nutritional composition"** means a mixture obtained by chemical and/or biological means, which can be chemically identical to the mixture naturally occurring in mammalian milks (i.e. the synthetic composition is not breast milk).

The expression **"infant formula"** as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A **"follow-up formula"** or **"follow-on formula"** is given from the 6th month onwards. It constitutes the principal liquid element in the progressively diversified diet of this category of person.

The expression **"baby food"** means a foodstuff intended for particular nutritional use by infants or young children during the first years of life.

The expression **"infant cereal composition"** means a foodstuff intended for particular nutritional use by infants or young children during the first years of life.

The term **"fortifier"** refers to liquid or solid nutritional compositions suitable for mixing with breast milk or infant formula.

The expressions **"days/weeks/months/years of life"** and **"days/weeks/months/years of birth"** can be used interchangeably.

The **"mother's milk"** should be understood as the breast milk or the colostrum of the mother.

An **"oligosaccharide"** is a saccharide polymer containing a small number (typically three to ten) of simple sugars (monosaccharides).

The term **"prebiotic"** means a substrate that is selectively utilized by host microorganisms conferring a health benefit' (Expert consensus document: The International Scientific Association for Probiotics and Prebiotics (ISAPP) consensus statement on the definition and scope of prebiotics, Nature Reviews Gastroenterology & Hepatology, 2017, 14, 491-502).

The term **"probiotic"** means live microorganisms that, when administered in adequate amounts, confer a health benefit on the host (FAO/WHO, 2002). The microbial cells are generally bacteria or yeasts.

All percentages are by weight unless otherwise stated.

### Process for producing complexes

The present invention provides a particular process for producing complexes of casein and a protein having an isoelectric point of a least 7. The obtained complexes can be soluble complexes or coacervates. Preferably the complexes are coacervates.

The process comprises a specific sequence of heating, holding and cooling steps. The complexes obtained by this specific process are particularly robust and can be maintained despite changes in the environment and can undergo harsh conditions, such as pH change, heating, application of mechanical stress, change in ionic strength and addition of surfactants, as will be shown in the examples.

Any protein having an isoelectric point of at least 7 can form complexes with casein using the process of the present invention. In a preferred aspect of the invention, the protein has an isoelectric point of at least 8, preferably at least 8.5, most preferably, an isolectric point of 8.9. Such proteins are preferred because the higher the isoelectric point, the more strongly cationic they will be at neutral pH and thus the more easily they will interact with caseins at natural pH.

Particularly advantageous proteins having an isoelectric point of at least 7 include lactoferrin, lactadherin and lysozyme. Such proteins are particularly preferred, owing to the health benefits that have been associated to their consumption. Lactoferrin for examples has been described as having a beneficial role in preventing, reducing and/or treating infections, modulating, promoting and/or supporting the immune response, promoting and/or supporting the growth of beneficial gut flora, promoting and/or supporting cognitive function, promoting and/or supporting healthy growth of an infant or young child; or promoting soft stools in an infant.

All types of caseins would work for the purpose of the present invention. β-Casein, κ-casein and α-s1 casein are however preferred for addition in products for infants and young children, such as infant formula, follow-on formula, infant cereals, baby food and growing-up milk, in so far as they are naturally present in human breast milk. wheras α-s2 casein is found in bovine milk but not in breast milk.

Casein, and in particular β-casein exhibits unique properties allowing the complexes to form. In particular the strongly hydrophilic N-terminal domain and the strongly hydrophobic C terminal domain of the β-casein sequence allow it to form surfactant like aggregates. This particular contrast in the β-casein sequence confers to this protein properties that are particularly useful for the purpose of the present invention. As one-tenth of the amino acids at the N-terminus of the protein contain one-third of the total charge, and with a pl of 5, a high density of negative charges are formed at the N-terminus at neutral pH, thus making it possible for this protein to strongly bind to proteins that are cationic at this pH.

In a preferred aspect, casein is in the form of micellar casein. Micellar casein is particularly advantageous over caseinate in nutritional compositions, such as infant formula, growing-up milks. Micellar casein has a different behaviour compared to caseinate, such as for example sodium caseinate. The process of the present invention is particularly advantageous in that it allows to form complexes with all types of casein as defined above, including micellar casein.

In preferred aspect of the invention, the protein having an isoelectric point above 7 is lactoferrin and the casein is β-casein.

In the first step of the process of the invention (step (a)), a solution is provided of the protein having an isoelectric point of at least 7 and casein. This solution is prepared in a way known to the person skilled in the art, by dissolving both proteins in an aqueous medium. In a preferred aspect of the invention, the solution provided in step a) comprises the protein having an isoelectric point of at least 7 and casein in a weight ratio of more than 1:10 to less than 10:1. In a preferred aspect of the invention, weight ratio of the protein having an isoelectric point of at least 7 to casein is of at least 1:9, preferably at least 1:8, more preferably at least 1:7, even more preferably at least 1:6 and most preferably at least 1:5. In another preferred aspect of the invention the weight ratio of the protein having an isoelectric point of at least 7 to casein is of at most 9:1, preferably at most 8:1, more preferably at least 7:1, even more preferably at least 6:1 and most preferably at most 5:1. For example the weight ratio of the protein having an isoelectric point of at least 7 to casein may advantageously be of 1:9 to 9:1, preferably of 1:8 to 8:1, more preferably of 1:7 to 7:1, even more preferably of 1:5 to 5:1. In particularly preferred embodiment, the weight ratio of the protein having an isoelectric point of at least 7 to casein is of 1:5 to 4:1, preferably 1:5 to 3:1, more preferably 1:5 to 2:1, most preferably 1:5 to 1:1.

Preferably the pH of the proteins solution is adjusted to a value in the range of 6.5 to 7.5. Preferably the pH is of 6.5 to 7.3, preferably it is of 6.5. to 7.2, preferably it is of 6.6 to 7.1, more preferably it is of 6.7 to 7.0, more preferably of 6.7 to 6.9, most preferably 6.8.

In a preferred aspect of the invention, the solution has a total protein content of 3 to 10% by weight, based on the total weight of the solution.

In an optional step, the proteins can be conditioned at the temperature of denaturation of the protein having an isoelectric point of at least 7 before further processing the solution. Such optional conditioning step is carried out by heating the protein solution at the temperature of denaturation of the protein having an isoelectric point of at least 7 and by holding the solution at that temperature for at least 2 minutes, preferably at least 3 minutes. Although this step is not necessary to form the complexes, their formation is promoted by the partial denaturation of the protein having an isoelectric point of at least 7 that is achieved during this conditioning phase. During this step unfolding of the structure of the protein has the effect of exposing different groups for interaction with casein. In a preferred aspect, such conditioning step is performed at a temperature of 60 to 70°C. Such temperature range is particularly adapted to lactoferrin. Most preferred temperature for the conditioning of lactoferrin is 65°C.

In the second essential step of the process, the solution is heated to a temperature in the range of 85 to 95°C, preferably in the range of 86 to 94°C, more preferably in the range of 87 to 93°C, more preferably in the range of 88 to 92°C, even more preferably in the range of 89 to 91°C and most preferably 90°C. The time needed to heat the solution to such temperature is not critical for the success of the formation of the complexes. As a matter of principle the longer the ramping-up time, the better the complex formation. The maximum time for the step of ramping-up the temperature is therefore determined by best cost/performance ratio. Such optimal duration of the ramping-up time may vary, for example depending of the volume that is processed and can be determined by the person skilled in the art by basic trial and error. Best results are however obtained when the temperature is ramped up over a period of 1 minute to 1 hour, preferably 1 to 30 minutes.

The solution is then held at such temperature over a period of at least 30 seconds, preferably at least 1 minute. Preferably the solution is not held at such temperature for more than 10 minutes, more preferably nor for more than 5 minutes.

After such holding period the temperature is ramped down to a temperature below 5°C. It is essential that the temperature is ramped down over a sufficiently long period of time to allow formation of the complexes. A period of at least 30 minutes is appropriate. Preferably the temperature is ramped down over a period of 1 hour. Preferably, the temperature is ramped down over a period of at most 10 hours, more preferably over a period of at most 5 hours, even more preferably over a period of at most 3 hours. Such limitation of the duration of the step of ramping down the process aims at keeping the process economically viable. The temperature is ramped down to a temperature of 5°C or less, preferably of less than 5°C, more preferably to a temperature of 4°C or less. Negative temperatures are however unsuitable during such holding step, as the aqueous solution of proteins would freeze and the process of forming the complexes would be stopped. Also the freezing would concentrate the ionic environment around the proteins leading to undesirable further protein denaturation or structural changes. The temperature is thus preferably ramped down to a temperature of 5 to 1 °C, preferably less than 5°C to 1°C, more preferably less than 5°C to 2°C, most preferably 4 to 2°C.

The ramping down of the temperature over an extended period of time, as described above, is particularly advantageous in that it makes it possible to make complexes from casein in all forms, and in particular also with micellar casein. The slow cooling is essential for breaking down the micelles and make the proteins available for interacting with the protein having an isoelectric point of at least 7. A process involving a sudden cooling is less advantageous than the present process as it is unsuitable for forming complexes from micellar casein and would only work with caseinate.

After ramping down the temperature to the above mentioned values, the solution is then held at such temperature to let time to finalize the formation of the complexes. The solution is preferably held at that temperature for at least 20 minutes, preferably for 30 minutes. Such minimum duration is necessary for the complexes to successfully form. The time the solution is held at such temperatures in not particularly limited. As will be evidenced in the examples the solution can be kept refrigerated for days. However, the duration of the holding time is limited in practice by economical consideration. Excessive duration of the holding step would make the process non-economically viable. It is thus preferred to hold the solution at such temperature for at most 1 day, preferably at most 10 hours, more preferably at most 5 hours, most preferably at most 3 hours.

### Complexes

Complexes, preferably coacervates, obtained or obtainable from the process of the present invention are different from the complexes obtained by standard process of consisting only adjusting the pH of the protein solution at a specific temperature, ionic strength and stoichiometric ratio. The successive steps of heating, cooling and holding the protein compositions at specific temperatures overs specific periods of time, as described above, lead to significant changes in the complex structure, which render the complexes more robust than prior art complexes to namely heating, pH modifications, mechanical stress and presence of surfactants. Therefore, complexes obtained or obtainable by the process according to any of the above-described embodiments is also an object of the present invention.

Preferred complexes according to the present invention are complexes, preferably coacervates, of lactoferrin and casein, even more preferably complexes of lactoferrin and β-casein, most preferably coacervates of lactoferrin and β-casein.

### Product comprising the complexes

The complexes of the present invention can advantageously be added in all kinds of products in which it is desirable to add casein and/or the protein having an isoelectric point of at least 7, such as for example lactoferrin, lactadherin and/or lysozyme.

The product can be any type of product in which the complexes can be incorporated, such as a product in the form of a food or beverage product, an animal feed product, a nutritional supplement for human or animal, a pharmaceutical composition or a cosmetic composition. The product may be in solid, liquid or semi-liquid form.

Food and beverage products include all products intended to be consumed orally by human beings, for the purpose of providing nutrition and/or pleasure. It can for example be a nutritional composition, such as for infants and/or young children, for a pregnant or lactating woman or a woman desiring to get pregnant, for individuals in need of a special nutrition due to an adverse health condition or for elderly people. More preferably, the nutritional composition is selected from infant formula, infant cereals, follow-up formula, growing-up milks and milk products for pregnant and lactating women or for women desiring to get pregnant. Other examples of food and beverage products include dairy products such as milk products or yogurts, soups, sauces, sweet and savoury snacks, powdered drinks and cereal products. Typically, dairy products advantageously comprises the complexes of the present invention, as casein is already naturally present in such ingredients.

The product can also be in the form of an animal food product or a nutritional supplement for animals. Preferably, the animal is a mammal. Examples of animals include primates (e.g., humans), cows, sheep, goats, horses, dogs, cats, rabbits, rats, mice, fish, birds and the like.

Nutritional supplements are typically present in the form of a liquid, a gel, a powder or a tablet or capsule. Powder supplements typically encompass supplements to be dissolved in water or to be sprinkled on food or in a beverage. Such supplements are intended to provide additional nutrients and/or a health benefit to the subject consuming it, as well as other beneficial ingredients, such as for example lactoferrin, lactadherin and/or lysozyme. A supplement according to the present invention can be used for providing nutrients and/or a health benefit to human beings, as well as to animals, as defined above. Nutritional supplements include for example powder supplements to be added to breast milk, for example for premature or low birth weight infants. It also includes supplements for pregnant or lactating woman or for woman desiring to get pregnant.

Pharmaceutical products include for example drops, syrups, powder, tablet or capsule products intended to treat of prevent an adverse medical condition in a subject in need thereof.

Cosmetic compositions are typically intended for an aesthetic effect on the body and may be for topical use or may be administered by oral route.

The product of the present invention preferably comprises the complexes of the present invention in a therapeutically effective amount.

In a preferred embodiment, the product of the present invention is an infant formula, an infant cereal composition, a follow-up formula or a growing-up milk comprising complexes, preferably coacervates, of lactoferrin and casein, more preferably complexes of lactoferrin and β-casein, most preferably coacervates of lactoferrin and β-casein. In such products, the complexes are preferably present in an amount providing from 0.5 to 10g, preferably 1 to 10g, more preferably 2 to 7g of lactoferrin per litre of the product.

All types of products according to the invention can be formulated and manufactured in accordance with the knowledge of the person skilled in the art. The complexes of the present invention are advantageously robust enough to be processed together with the other ingredients of the products.

For example, in the manufacture of a spray-dried product, such as an infant formula, a growing-up milk or a follow-up formula in powder form, the complexes are robust enough to be added in the wet mix and spray-dried together with the other ingredients of the product. This is advantageous from a process economy point of view, as aseptic dosing and dry mixing of sensitive proteins like lactoferrin, lactadherin and or lysozyme is not needed. In addition, incorporation of the complexes in the wet mix is advantageous from a product structure point of view. In particular, the complexes will be admixed in a homogeneous way with the other ingredients. In contrast, dry mixed powders may lead to inhomogeneous products due difference in the properties of the admixed powders such as for example difference in density or particle size. Inhomogeneity may lead to inaccurate dosage of the proteins.

Therefore, a process of making a product selected from an infant formula, a follow-up formula or a growing-up milk in powder form comprising preparing a product concentrate comprising complexes according to the present invention and spray-drying said product concentrate is also an object of the present invention. Preparing the wet mix and spray-drying are carried out in accordance with the general knowledge of the person skilled in the art.

In another aspect the invention provides a process for making a product selected from a liquid infant formula, follow-up formula or growing-up milk comprising admixing the complexes of the present invention to the liquid product base and aseptically processing the product base comprising the complexes. The preparation of the liquid product base and the aseptic processing are carried out in accordance with the general knowledge of the person skilled in the art.

### First medical use

The complexes, preferably the coacervates, or the product comprising complexes, preferably comprising coacervates, as described above, can advantageously be used in therapy. Thus the invention also provides for such complexes and such products for use in therapy. In a particularly preferred aspect the invention provides for complexes, preferably coacervates of lactoferrin, lactadherin or lyzozyme and casein and products comprising such complexes or coacervates for use in therapy. Even more preferably such complexes are complexes, preferably coacervates of lactoferrin and casein, preferably of lactoferrin and β-casein.

Therapy is intended here as the curing or prevention of a disease or malfunction of the body and also covers prophylactic treatment, i.e. prevention of an adverse medical condition. Therapy is also intended here to include human and animal therapy.

In other words, the present invention relates to a method for treating a subject comprising administering to the subject a therapeutically effective amount of a complex or of a product according to the present invention.

In another embodiment the invention relates to the use of complexes or of a product according to the present invention for the manufacture of a medicament.

### Further medical uses

The invention provides complexes, preferably coacervates, of lactoferrin and casein or a product comprising such complexes, as described above, for use in the following specific therapeutic methods:
a) preventing, reducing and/or treating infections;
b) modulating, promoting and/or supporting the immune response;
c) promoting and/or supporting the growth of beneficial gut flora;
d) promoting and/or supporting cognitive function;
e) promoting and/or supporting healthy growth of an infant or young child; or
f) promoting soft stools in an infant.

In a preferred embodiment, the invention provides complexes, preferably coacervates, of lactoferrin and β-casein or a product comprising such complexes, as described above, for use for the following specific therapeutic methods:
a) preventing, reducing and/or treating infections;
b) modulating, promoting and/or supporting the immune response;
c) promoting and/or supporting the growth of beneficial gut flora;
d) promoting and/or supporting cognitive function;
e) promoting and/or supporting healthy growth of an infant or young child; or
f) promoting soft stools in an infant.

In a preferred embodiment, the invention provides an infant formula, a follow-on formula or a growing-up milk comprising complexes, preferably coacervates, of lactoferrin and β-casein, as described above, for use for the following specific therapeutic methods:
a) preventing, reducing and/or treating infections;
b) modulating, promoting and/or supporting the immune response;
c) promoting and/or supporting the growth of beneficial gut flora;
d) promoting and/or supporting cognitive function;
e) promoting and/or supporting healthy growth of an infant or young child; or
f) promoting soft stools in an infant.

The present invention will now be described in further details by the way of the following examples.

### Example 1: Preparation and detection of complexes according to the present invention

Stock solutions of lactoferrin (origin: Westland Milk Products (HY03; 98.9% protein; from Westand) and micellar β-casein (origin: Ultranor Beta (Y044; 75% protein; from Kerry) at a protein concentration of 5.5% w/w were prepared and allowed to solubilise for 2 hours at speed 3 using a magnetic stirrer on a Stuart Stirrer Multiposition SB 162-3. In order to make a mixture at a 1:1 (β-cn : Lf) weight ratio, appropriate volumes of each stock solution were then weighed out and mixed together for 2 minutes using a Pasteur Pipette. The pH was adjusted then to 6.8 using 0.1M HCl/ NaOH and the solution was directly taken to the rheometer (AR2000ex, from TA Instruments) for heat treatment. An amount of 28g (± 0.05g) of the mixture was weighed into the sample cup of the rheometer. The sample was then subjected to a Heat-Cool-Hold SPC treatment as described in Table 1 below.

**Table 1: Heat-Cool-Hold SPC treatment log**

| | | |
|---|---|---|
| 1. | Conditioning Step | Initial Temperature: 65.0 °C |
| | | Normal Force: 0 |
| | | Pre Shear angular velocity : 16.78 (rad/s) for 1 minute |
| | | Equilibration: 2 minutes |
| 2. | Peak Hold Step 1 | Temperature: 65°C |
| | | Duration: 1 minute and 20 seconds |
| | | Angular velocity: 16.78 (rad/s) |
| 3. | Temperature Ramp Step 1 | Ramp from 65 to 87°C |
| | | Ramp duration: 2 minutes and 4 seconds |
| | | Angular velocity: 16.78 (rad/s) |
| 4. | Temperature Ramp Step 2 | Ramp from 87 to 90°C |
| | | Ramp Duration : 1 minutes and 20 seconds |
| | | Shear Stress (Pa): 0.1080 |
| 5. | Peak Hold Step 2 | Temperature : 90°C |
| | | Duration: 1 minute |
| | | Angular velocity: 16.78 (rad/s) |
| 6. | Temperature Ramp Step 3 | Ramp from 90 to 4°C |
| | | Ramp Duration: 1 hour |
| | | Angular velocity : 16.78 (rad/s) |
| 7. | Peak Hold Step 3 | Temperature: 4°C |
| | | Duration: 30 minutes |
| | | Angular Velocity: 16.78 (rad/s) |

The obtained solution was analyzed by confocal laser scanning microscopy (CLSM) using a Leica TCS SP5 confocal laser scanning microscope (Leica Microsystems, Heildelberg GmbH, Mannheim, Germany). A small amount of sample was placed on the slide. Samples were dyed by pipetting 10µL of Nile green dye (0.001g/100 mL) onto the surface of the sample. The cover slip was placed on top. 3D projections of z-stacks of the complexes were provided. Z-stacks involve the imaging of numerous planes of the samples, in which a 3D image can be rendered

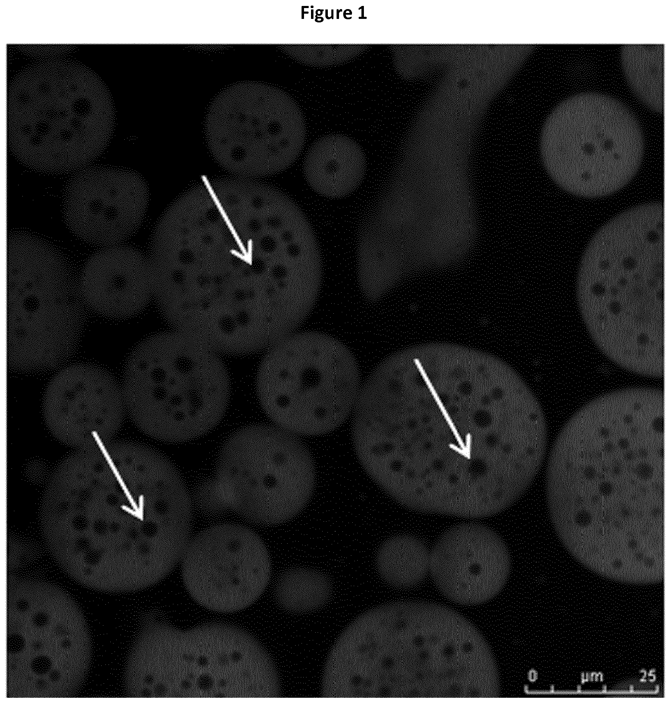

The CLSM analysis revealed that complexes of β-casein and lactoferrin were formed. Figure 1 depicts a still image taken through the Z-stack of the sample using the 63 objective lens. Coacervates form spherical particles with uniform shape. The coacervates are also characterized by a porous structure, with numerous vacuoles, which are highlighted on the figure by the white arrows.

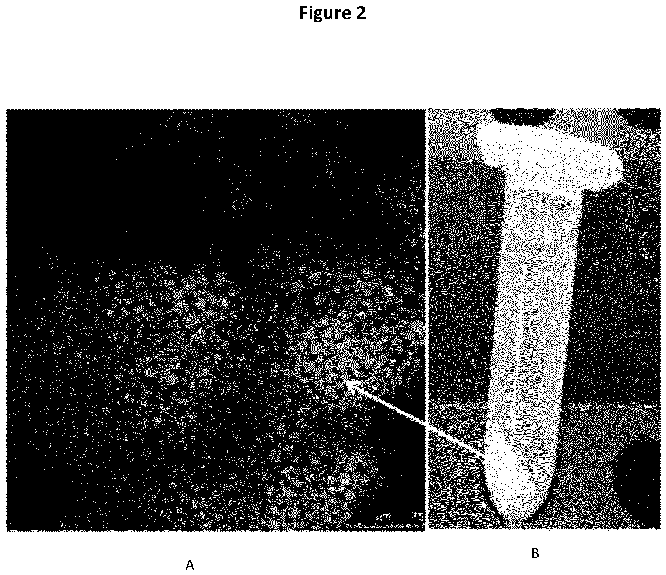

The sample was then centrifuged at 8000 RCF for 5 minutes at 20°C. A clear separation between sediment layer (coacervate) and the supernatant layer (dilute equilibrium phase) could be seen, as shown on Figure 2B. Both the sediment (pellet) and the supernatant were analyzed by CLSM analysis as described above. Figure 2A shows the CLSM image of the pellet with the 20 objective lense. The coacervates can again be seen clearly as spherical particles.

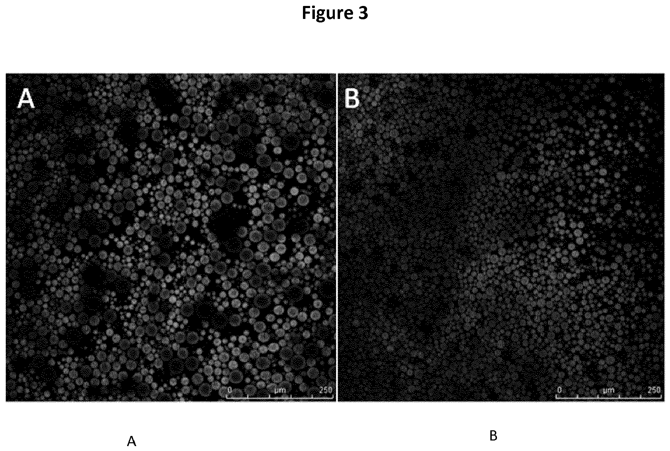

The composition was then subjected to 24 hours refrigeration at 4°C. The sediment layer containing the coacervates was analyzed by CLSM before (Figure 3A) and after (Figure 3B) the refrigeration day. Although both images comprise of distinct and clearly formed coacervates, there is a clear difference in size, indicating that coacervates decrease in size as holding time at low temperature increases. Such decrease in size is associated with reduced occurrence of the vacuoles within the structure of the coacervates.

As will be shown in the following comparative examples, the formation of the coacervates is the result of the combination of subsequent steps of heating, slow cooling and holding at low temperature. Subjecting the β-casein and lactoferrin composition to slow cooling and holding at 4°C alone, as well as the heat treatment alone, were not successful in obtaining coacervates.

### Example 2: Lactoferrin and β-casein composition without heat or cool treatment (comparative)

Stock solutions of lactoferrin (origin: Westland Milk Products (HY03; 98.9% protein) and micellar β-casein (origin: Ultranor Beta (Y044; 75% protein) at a protein concentration of 5.5% w/w were prepared by dissolving the protein in water. A solution of lactoferrin and β-casein at a 1:1 weight ratio was prepared by admixing the two stock solutions. The pH was adjusted to 6.8. Such solution was conditioned at 65°C for approximately 3 minutes.

The obtained solution was analyzed by confocal laser scanning microscopy (CLSM). A small amount of sample was placed on the slide. Samples were dyed by pipetting 10µL of Nile green dye (0.001 g/100 mL) onto the surface of the sample. The cover slip was placed on top.

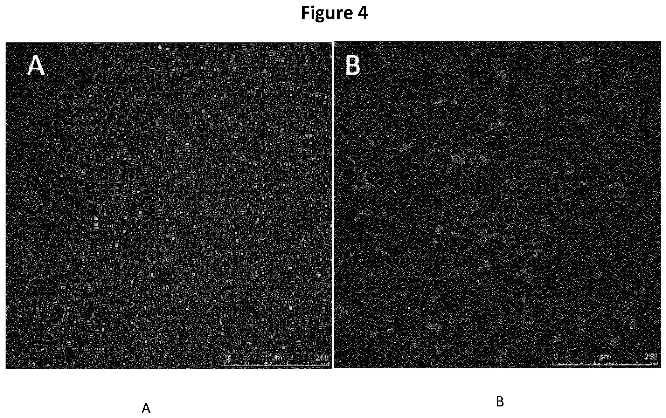

No complexes of lactoferrin and β-casein were formed. In particular, as can be seen on Figure 4, no coacervates could be found in the sample.

### Example 3: Slow cooling of lactoferrin and β-casein composition (comparative)

Stock solutions of lactoferrin (origin: Westland Milk Products (HY03; 98.9% protein) and micellar β-casein (origin: Ultranor Beta (Y044; 75% protein) at a protein concentration of 5.5% w/w were prepared by dissolving the protein in water. A solution of lactoferrin and β-casein at a 1:1 weight ratio was prepared by admixing the two stock solutions. The pH was adjusted to 6.8. Such solution was conditioned at 65°C for approximately 3 minutes.

The temperature was then ramped down from 65°C to 4°C over 1 hour. The solution was then held at 4°C for 30 minutes. The obtained solution was analyzed by confocal laser scanning microscopy (CLSM). A small amount of sample was placed on the slide. Samples were dyed by pipetting 10µL of Nile green dye (0.001g/100 mL) onto the surface of the sample. The cover slip was placed on top.

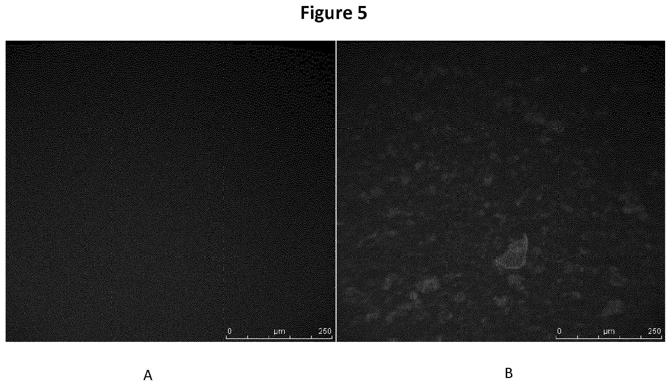

No complexes of lactoferrin and β-casein were formed. In particular, as can be seen on Figure 5, no coacervates could be found in the sample.

### Example 4: Heat treatment of lactoferrin and β-casein composition (comparative)

Stock solutions of lactoferrin (origin: Westland Milk Products (HY03; 98.9% protein) and micellar β-casein (origin: Ultranor Beta (Y044; 75% protein) at a protein concentration of 5.5% w/w were prepared by dissolving the protein in water. A solution of lactoferrin and β-casein at a 1:1 weight ratio was prepared by admixing the two stock solutions. The pH was adjusted to 6.8. Such solution was conditioned at 65°C for approximately 3 minutes.

The temperature was then ramped up from 65°C to 90°C. The solution was then held at 90°C for 1 minute and the temperature was then ramped down from 90 to 10°C over 5 minutes. It was then held at 10°C for 5 minutes. The obtained solution was analyzed by confocal laser scanning microscopy (CLSM). A small amount of sample was placed on the slide. Samples were dyed by pipetting 10µL of Nile green dye (0.001g/100 mL) onto the surface of the sample. The cover slip was placed on top.

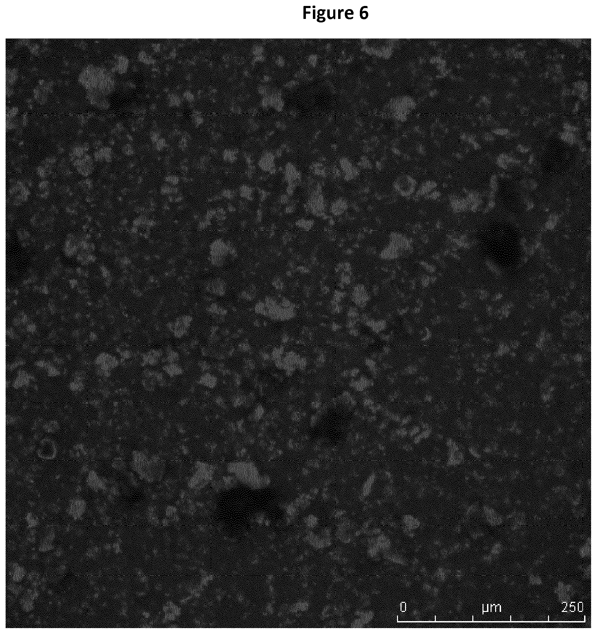

No complexes of lactoferrin and β-casein were formed. In particular, as can be seen on Figure 6, no coacervates could be found in the sample.

### Example 5: Assessment of the robustness of the complexes

The robustness of the coacervates produced as described in Example 1 was evaluated by subjecting such coacervates to diverse treatments and subsequently assessing if coacervates were still present on light microscope images..

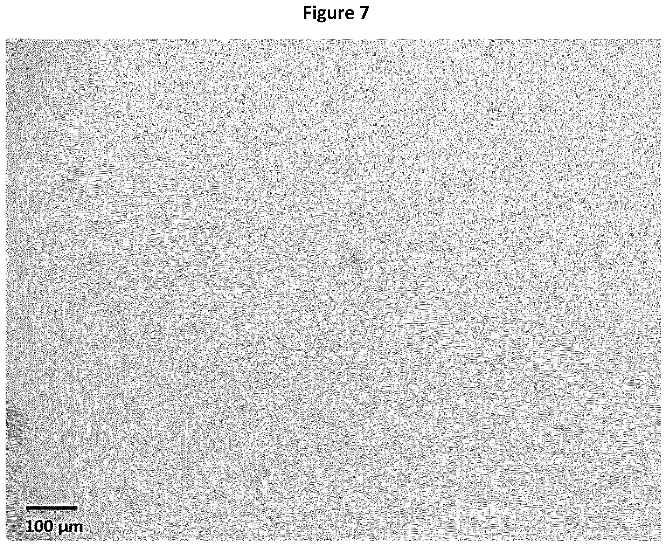

In a first trial, the coacervate pellet obtained in Example 1 was diluted in a 0.1% solution of sodium dodecyl sulphate (SDS). Even after being contacted with this ionic surfactant, Figure 7 shows that the coacervates remained stable.

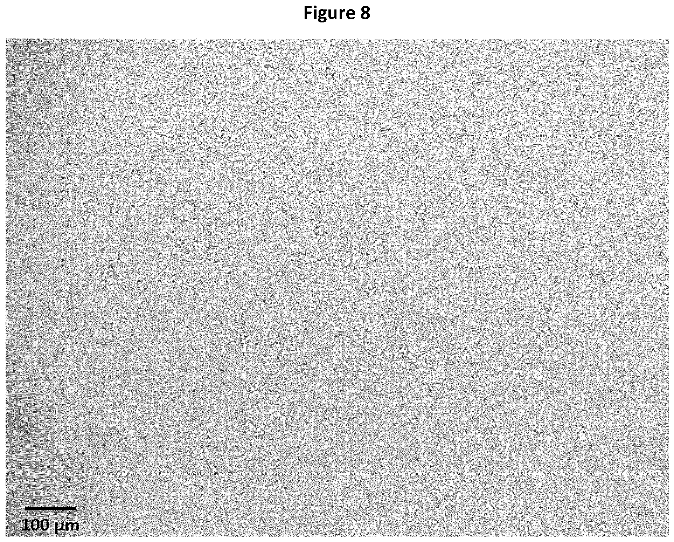

In a second experiment, the coacervate pellet obtained in Example 1 was diluted with 0.02N HCL and then centrifuged at 20,000 RCF for 20 minutes at 4°C. Despite the acidic treatment and centrifugation, the coacervates remained stable, as can be seen from Figure 8, depicting the coacervates present in the pellet.

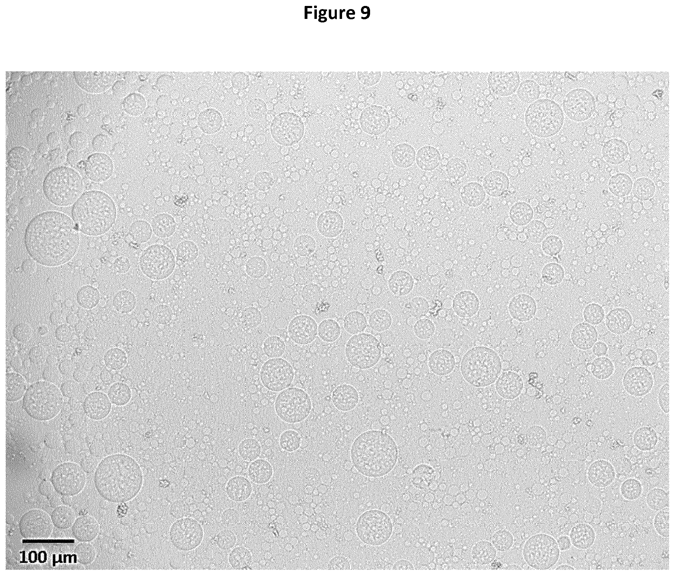

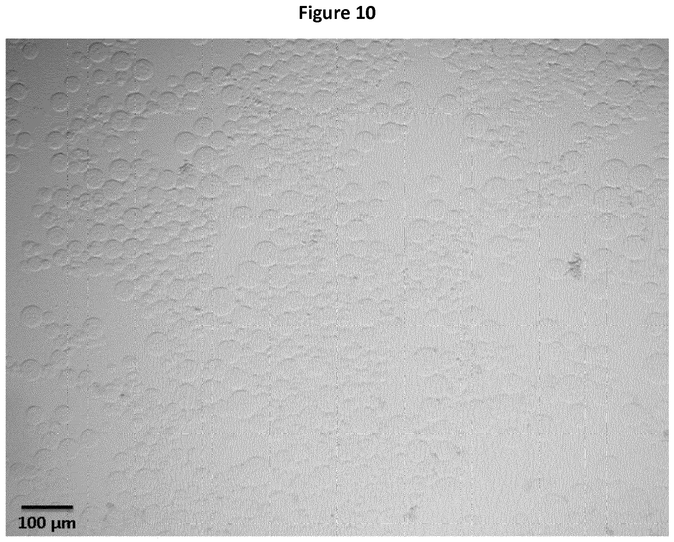

In a third experiment, the stability of the coacervates in acidic medium was assessed by diluting the coacervate pellet obtained in Example 1 in water and adjusting the pH to 3.5. Figure 9 shows that the coacervates are still present. Figure 10 shows also that coacervates are also retained after subsequent heat treatment at 70°C for 15 minutes at pH 3.5.

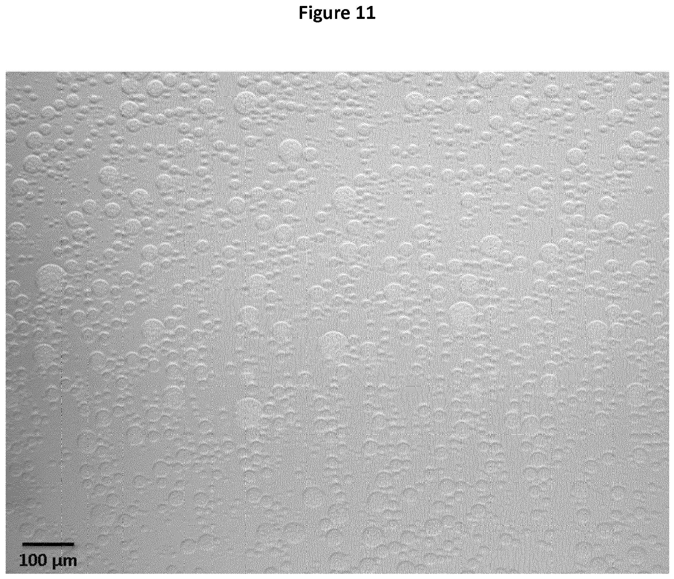

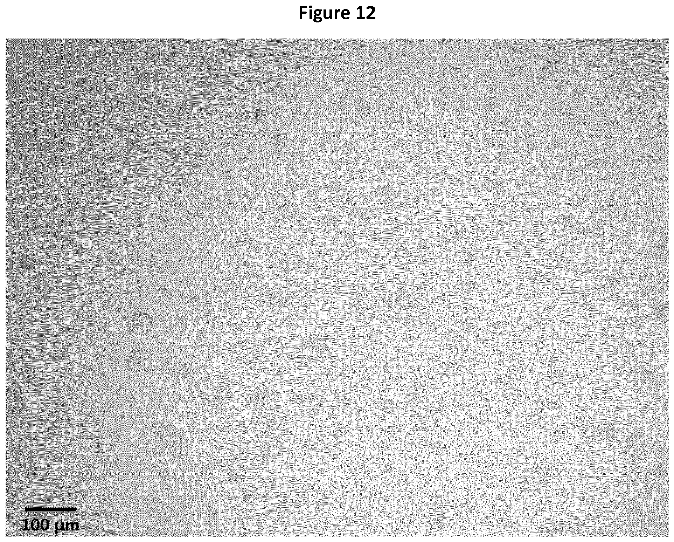

In a fourth experiment, the effect of basic pH has been investigated. The coacervates pellet obtained in Example 1 was diluted in water and the pH was adjusted to 10. Figure 11 shows that the coacervates remained stable. Figure 12 shows that this is still the case when the coacervates are heated to 70°C for 15 minutes after pH adjustment.

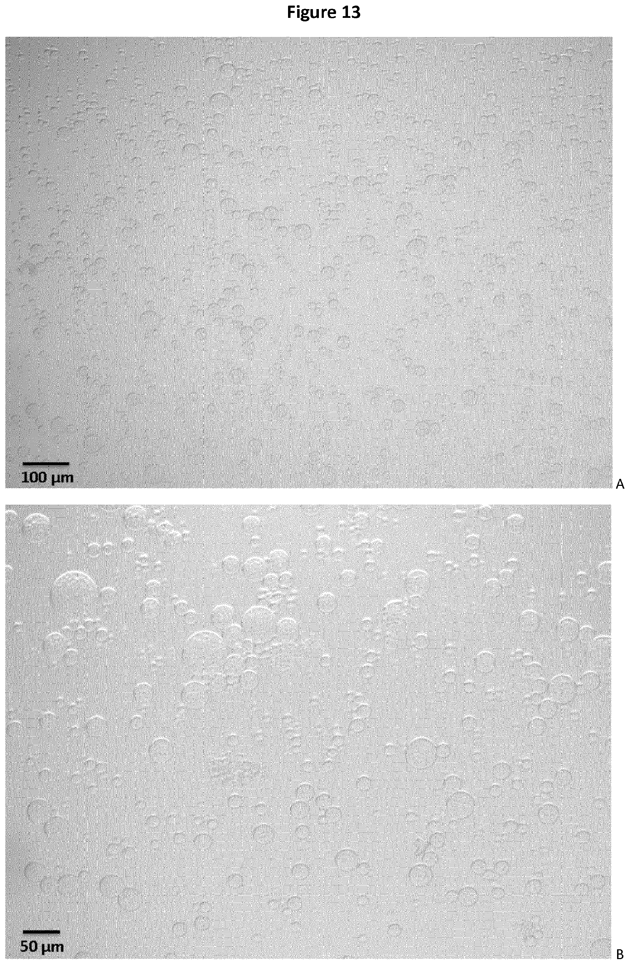

In a fifth experiment, the effect of ionic strength on the stability of the coacervates has been assessed by diluting the coacervate pellet obtained in Example 1 in 100 mM NaCl. The coacervates also remained stable under such conditions, as shown on Figure 13. The coacervates are enlarged 10 times on Figure 13A and 20 times on Figure 13B.

The results of these experiments show that the coacervates produced with the process of the present invention are substantially more stable than coacervates formed as described in the prior art. Indeed, such prior art coacervates that are formed by adjusting the pH of a lactoferrin and β-casein solution, have been described as being unstable, namely when heated or when subjected to a pH change. In the present example, we show that the coacervates of the present invention surprisingly remain stable in a broad range of pH and can withstand heating, harsh mechanical stress and do not dissolve in the presence of surfactants.

### Example 6: Effect of lactoferrin and β-casein weight ratio on formation of the complexes

Example 1 was repeated starting from a solution of lactoferrin and β-casein at a 1:5 weight ratio and with a solution of lactoferrin and β-casein at a 1:10 weight ratio. Except for the change of such ratio, the process was identical to that described in Example 1.

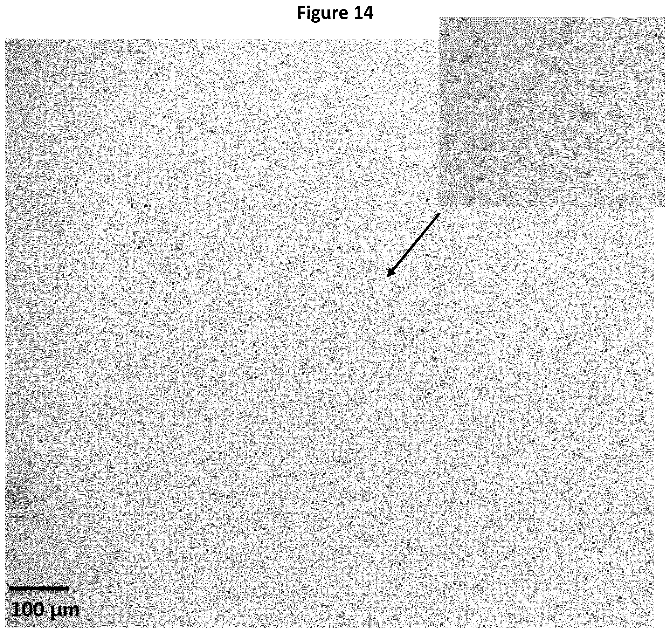

It is visible on Figure 14 that coacervates are formed with a lactoferrin to β-casein weight ratio of 1:5, even though they are of a smaller size than those obtained with a 1:1 weight ratio. On Figure 14, the small picture shows a 4x zoom of the area designated by the black arrow.

To the contrary, with a lactoferrin to β-casein weight ratio of 1:10 no coacervates were formed.

## Claims

1. A process for producing complexes of a protein having an isoelectric point of at least 7 with casein comprising the steps of:
a. providing a solution of a protein having an isoelectric point of at least 7 and casein at a pH in the range of 6.5 to 7.5;
b. ramping up the temperature from the denaturation temperature of the protein having an isoelectric point of 7 or more to a temperature in the range of 85 to 95°C;
c. holding the solution at a temperature of 85 to 95°C for at least 30 seconds; and
d. ramping down the temperature from 85-95°C to a temperature below 5°C over a period of at least 30 minutes.
e. holding the solution at a temperature below 5°C for at least 15 minutes.

2. A process according to claim 1, wherein the process is a process of making coacervates of a protein having an isoelectric point of at least 7 with casein.

3. A process according to claim 1 or 2, wherein a step of conditioning the solution of step a) at the denaturation temperature of the protein having an isoelectric point of at least 7 for at least 2 minutes is carried out before the ramping-up of the temperature.

4. A process according to claim any one of the preceding claims, wherein the protein has an isoelectric point of at least 8, preferably at least 8.5, most preferably 8.9.

5. A process according to any one of the preceding claims, wherein the casein is α-casein or β-casein, preferably β-casein.

6. A process according to any one of the preceding claims, wherein the solution provided in step a) comprises the protein having an isoelectric point of at least 7 and casein in a weight ratio of more than 1:10 and less than 10:1, preferably a weight ratio of 1:5 to 5:1.

7. A process according to any one of the preceding claims, wherein the solution provided in step a) has a pH of 6.6 to 7.4.

8. A process according any one of the preceding claims, wherein the solution is held at a temperature of 2 to 5°C after ramping down the temperature.

9. A process according to any one of the preceding claims, wherein the protein having an isoelectric point of at least 7 is selected from lactoferrin, lactadherin and lysozyme.

10. A process according to claim 9, wherein the protein having an isoelectric point of at least 7 is lactoferrin.

11. A complex of a protein having an isoelectric point of 7 or more with β-casein, obtainable or obtained by the process of any of claims 1 to 10.

12. A complex according to claim 11, wherein such complex is a coacervate.

13. A product comprising the complex of claim 11 or 12.

14. A complex obtained or obtainable by the process of claim 9 or 10 or a product comprising such complex, for use in therapy.

15. A complex obtained or obtainable by the process of claim 10 or a product comprising such complex, for use in a method of
a) preventing, reducing and/or treating infections;
b) modulating, promoting and/or supporting the immune response;
c) promoting and/or supporting the growth of beneficial gut flora;
d) promoting and/or supporting cognitive function;
e) promoting and/or supporting healthy growth of an infant or young child; or
f) promoting soft stools in an infant.

## Patentansprüche

1. Vorgang zum Erzeugen von Komplexen eines Proteins, das einen isoelektrischen Punkt von mindestens 7 mit Kasein aufweist, umfassend die Schritte:
a. Bereitstellen einer Lösung eines Proteins, das einen isoelektrischen Punkt von mindestens 7 und Kasein bei einem pH-Wert in dem Bereich von 6,5 bis 7,5 aufweist;
b. Steigern der Temperatur von der Denaturierungstemperatur des Proteins, das einen isoelektrischen Punkt von 7 oder mehr aufweist, auf eine Temperatur in dem Bereich von 85 bis 95 °C;
c. Halten der Lösung bei einer Temperatur von 85 bis 95 °C für mindestens 30 Sekunden; und
d. Steigern der Temperatur von 85-95 °C auf eine Temperatur unter 5 °C über einen Zeitraum von mindestens 30 Minuten.
e. Halten der Lösung bei einer Temperatur unter 5 °C für mindestens 15 Minuten.

2. Vorgang nach Anspruch 1, wobei der Vorgang ein Vorgang zum Herstellen von Koazervaten eines Proteins ist, das einen isoelektrischen Punkt von mindestens 7 aufweist, mit Kasein.

3. Vorgang nach Anspruch 1 oder 2, wobei ein Schritt der Konditionierung der Lösung von Schritt a) bei der Denaturierungstemperatur des Proteins, das einen isoelektrischen Punkt von mindestens 7 für mindestens 2 Minuten aufweist, vor dem Steigern der Temperatur ausgeführt wird.

4. Vorgang nach einem der vorstehenden Ansprüche, wobei das Protein einen isoelektrischen Punkt von mindestens 8, vorzugsweise mindestens 8,5, am stärksten bevorzugt 8,9 aufweist.

5. Vorgang nach einem der vorstehenden Ansprüche, wobei das Kasein α-Kasein oder β-Kasein, vorzugsweise β-Kasein ist.

6. Vorgang nach einem der vorstehenden Ansprüche, wobei die in Schritt a) bereitgestellte Lösung das Protein, das einen isoelektrischen Punkt von mindestens 7 aufweist und Kasein in einem Gewichtsverhältnis von mehr als 1:10 und weniger als 10:1, vorzugsweise einem Gewichtsverhältnis von 1:5 bis 5:1, umfasst.

7. Vorgang nach einem der vorstehenden Ansprüche, wobei die in Schritt a) bereitgestellte Lösung einen pH-Wert von 6,6 bis 7,4 aufweist.

8. Vorgang nach einem der vorstehenden Ansprüche, wobei die Lösung bei einer Temperatur von 2 bis 5 °C nach dem Herunterfahren der Temperatur gehalten wird.

9. Vorgang nach einem der vorstehenden Ansprüche, wobei das Protein, das einen isoelektrischen Punkt von mindestens 7 aufweist, aus Lactoferrin, Lactadherin und Lysozym ausgewählt ist.

10. Vorgang nach Anspruch 9, wobei das Protein, das einen isoelektrischen Punkt von mindestens 7 aufweist, Lactoferrin ist.

11. Komplex eines Proteins, das einen isoelektrischen Punkt von 7 oder mehr mit β-Kasein aufweist, erhältlich oder erhalten durch den Vorgang nach einem der Ansprüche 1 bis 10.

12. Komplex nach Anspruch 11, wobei ein solcher Komplex ein Koazervat ist.

13. Erzeugnis, umfassend den Komplex nach Anspruch 11 oder 12.

14. Komplex, der durch den Vorgang nach Anspruch 9 oder 10 erhalten wird, oder ein Erzeugnis, umfassend einen solchen Komplex, zur Verwendung in der Therapie.

15. Komplex, erhalten oder erhältlich durch den Vorgang nach Anspruch 10 oder ein Erzeugnis, umfassend einen solchen Komplex, zur Verwendung in einem Verfahren zum
a) Verhindern, Reduzieren und/oder Behandeln von Infektionen;
b) Modulieren, Fördern und/oder Unterstützen der Immunantwort;
c) Fördern und/oder Unterstützen des Wachstums von vorteilhafter Darmflora;
d) Fördern und/oder Unterstützen der kognitiven Funktion;
e) Fördern und/oder Unterstützen des gesunden Wachstums eines Säuglings oder Kleinkinds; oder
f) Fördern von weichem Stuhl in einem Säugling.

## Revendications

1. Procédé de production de complexes d'une protéine ayant un point isoélectrique d'au moins 7 avec de la caséine comprenant les étapes consistant à :
a. fournir une solution d'une protéine ayant un point isoélectrique d'au moins 7 et de caséine à un pH dans la plage de 6,5 à 7,5 ;
b. augmenter progressivement la température à partir de la température de dénaturation de la protéine ayant un point isoélectrique de 7 ou plus jusqu'à une température dans la plage de 85 à 95 °C ;
c. maintenir la solution à une température de 85 à 95 °C pendant au moins 30 secondes ; et
d. réduire progressivement la température de 85 à 95 °C jusqu'à une température en dessous de 5 °C sur une période d'au moins 30 minutes.
e. maintenir la solution à une température en dessous de 5 °C pendant au moins 15 minutes.

2. Procédé selon la revendication 1, le procédé étant un procédé de fabrication de coacervats d'une protéine ayant un point isoélectrique d'au moins 7 avec de la caséine.

3. Procédé selon la revendication 1 ou 2, dans lequel une étape de conditionnement de la solution de l'étape a) à la température de dénaturation de la protéine ayant un point isoélectrique d'au moins 7 pendant au moins 2 minutes est effectuée avant l'augmentation progressive de la température.

4. Procédé selon la revendication de l'une quelconque des revendications précédentes, dans lequel la protéine a un point isoélectrique d'au moins 8, de préférence au moins 8,5, le plus préférablement 8,9.

5. Procédé selon l'une quelconque des revendications précédentes, dans lequel la caséine est de l'α-caséine ou de la β-caséine, de préférence de la β-caséine.

6. Procédé selon l'une quelconque des revendications précédentes, dans lequel la solution fournie à l'étape a) comprend la protéine ayant un point isoélectrique d'au moins 7 et de la caséine dans un rapport pondéral supérieur à 1:10 et inférieur à 10:1, de préférence un rapport pondéral de 1:5 à 5:1.

7. Procédé selon l'une quelconque des revendications précédentes, dans lequel la solution fournie à l'étape a) a un pH de 6,6 à 7,4.

8. Procédé selon l'une quelconque des revendications précédentes, dans lequel la solution est maintenue à une température de 2 à 5 °C après réduction progressive de la température.

9. Procédé selon l'une quelconque des revendications précédentes, dans lequel la protéine ayant un point isoélectrique d'au moins 7 est choisie parmi lactoferrine, lactadhérine et lysozyme.

10. Procédé selon la revendication 9, dans lequel la protéine ayant un point isoélectrique d'au moins 7 est lactoferrine.

11. Complexe d'une protéine ayant un point isoélectrique de 7 ou plus avec de la β-caséine, pouvant être obtenu ou obtenu par le procédé selon l'une quelconque des revendications 1 à 10.

12. Complexe selon la revendication 11, dans lequel un tel complexe est un coacervat.

13. Produit comprenant le complexe selon la revendication 11 ou 12.

14. Complexe obtenu ou pouvant être obtenu par le procédé selon la revendication 9 ou 10 ou produit comprenant un tel complexe, pour utilisation en thérapie.

15. Complexe obtenu ou pouvant être obtenu par le procédé selon la revendication 10 ou produit comprenant un tel complexe, pour utilisation dans un procédé de
a) prévention, réduction et/ou traitement d'infections ;
b) modulation, promotion et/ou soutien de la réponse immune ;
c) promotion et/ou soutien de la croissance de la flore intestinale bénéfique ;
d) promotion et/ou soutien de la fonction cognitive ;
e) promotion et/ou soutien de la croissance en bonne santé d'un nourrisson ou jeune enfant ; ou
f) promotion des selles molles chez un nourrisson.
